# EUROPÄISCHE PATENTSCHRIFT

(11) **EP 0 661 382 B1**
(45) Veröffentlichungstag und Bekanntmachung des Hinweises auf die Patenterteilung: **01.03.2000**
(21) Anmeldenummer: 94118336.0
(22) Anmeldetag: 22.11.1994
(51) Int. Cl.: C12P 7/26

(54) **Mikrobielles Verfahren zur Herstellung von Dihydroxyaceton**
Microbial process for the preparation of dihydroxyacetone
Procédé microbienne pour la préparation de dihydroxyacetone

(30) Priorität: 03.12.1993 DE 4341238
(43) Veröffentlichungstag der Anmeldung: 05.07.1995
(73) Patentinhaber: MERCK PATENT GmbH, 64293 Darmstadt (DE)
(72) Erfinder: Ohrem, Hans-Leonard, D-64331 Weiterstadt (DE)

(56) Entgegenhaltungen:
- DD-A- 253 836
- US-A- 2 948 658
- US-A- 4 076 589
- ANNALI DI MICROBIOLOGIA ED ENZIMOLOGIA, Bd. 15, Nr. 6, 1965 Seiten 203-207, XP 000566842 FRANCO MARINI 'Transformazione del glicerolo in diidrossiacetone con Acetobacter suboxydans'
- JOURNAL OF BIOCHEMICAL AND MICROBIOLOGICAL TECHNOLOGY AND ENGINEERING , Bd. III, Nr. 4, 1961 Seiten 351-355, XP 000566771 S.R. GREEN ET AL. 'Dihydroxyacetone: Production and uses'

## Beschreibung

Die Erfindung betrifft ein mikrobielles Verfahren zur Herstellung von Dihydroxyaceton, wobei die Umsetzung von Glycerin zu Dihydroxyaceton mit Hilfe eines dehydrogenaseaktiven Mikroorganismus bei einem durch Zugabe von Calciumhydroxid-Suspension konstant gehaltenen pH-Wert zwischen 3.8 und 4.8, vorzugsweise von 4.2, durchgeführt wird.

Die Gewinnung von Dihydroxyaceton aus Glycerin mit geeigneten Mikroorganismen ist seit langem allgemein bekannt. Da Dihydroxyaceton ein wertvolles Ausgangsprodukt für die chemische und pharmazeutische Industrie darstellt, besteht ein permanentes Interesse, die Verbindung auf möglichst einfache und wirtschaftliche Weise im großtechnischen Maßstab zur Verfügung zu stellen. Entsprechend wurden im Laufe der Jahre Variationen an den Kultivierungs- und Fermentationsbedingungen vorgenommen mit dem Ziel, ständig die Ausbeute zu erhöhen (z.B. US 2,948,658, US 4,076,589, DD 230 713, DD 253 836). Insbesondere wurde der Tatsache Aufmerksamkeit geschenkt, daß sowohl der Menge an Edukt (Glycerin) als auch an synthetisiertem Produkt (Dihydroxyaceton) offensichtlich eine entscheidende Rolle beigemessen werden muß, da beide Verbindungen, wenn sie in größeren Konzentrationen in der Fermentationsbrühe vorliegen, Wachstum und/oder Produktivität des eingesetzten Mikroorganismus in negativer Weise beeinflussen (z.B. Claret et al. (1992), Current Microbiol. 25(3), 149). Die Fermentationsbrühen des Standes der Technik enthalten Glycerin zumeist in 5-20%iger Konzentration, das in einigen wenigen Fällen zugefüttert wird. Zusatzstoffe wie Glucose, Sorbit, Hefe-Extrakte, Maisquellwasser und bei der Zellkultivierung übliche Salze sind in Kenzentrationen in der Regel zwischen unter 1 % und bis zu 15 % im Fermentationsansatz enthalten. Der pH-Wert während der mikrobiellen Synthese von Dihydroxyaceton variiert in der Regel zwischen 5 und 7; nur in einigen Fällen, insbesondere bei denen Acetobacter als Mikroorganismus eingesetzt wurde, wird ein pH-Wert zwischen 3 und 5 beschrieben (DD 253 836). Die im Stand der Technik angegebenen Ausbeuten an Dihydroxyaceton varriieren durchschnittlich zwischen 10 und 150 g/l bei einer Kultivierungs- bzw. Reaktionszeit von 20 bis 70 h.

Es wurde nun gefunden, daß auf einfache Weise die Ausbeute an Dihydroxyaceton deutlich gesteigert werden kann, wenn die mikrobielle Umsetzung bei einem konstant gehaltenen pH-Wert zwischen 3.8 und 4.8 durchgeführt wird, wobei die Einhaltung des pH-Wertes durch Titration mit Calciumhydroxid-Suspension erreicht wird. Die Verwendung von Calciumhydroxid hat erfindungsgemäß gegenüber Alkali- oder anderer Erdalkalilaugen klare Vorteile.

Gegenstand der Erfindung ist somit ein Verfahren zur Herstellung von Dihydroxyaceton durch Dehydrierung von Glycerin mittels dehydogenaseaktiver Mikroorganismen, das dadurch gekennzeichnet ist, daß man die Reaktion durch Titration mittels Calciumhydroxid-Suspension bei einem konstant gehaltenen pH-Wert, ausgewählt in einem Bereich zwischen 3.8 und 4.8, durchführt. In einer bevorzugten Ausführungsform erfolgt die Umsetzung bei einem pH-Wert von 4.2.

Weiterhin wurde gefunden, daß die Ausbeuten besonders hoch sind, wenn die Glycerinkonzentration konstant gehalten wird.

Gegenstand der Erfindung ist somit auch ein entsprechendes Verfahren, das dadurch gekennzeichnet ist, daß der Glyceringehalt durch Zudosierung konstant zwischen 1 und 3% (w/v) gehalten wird.

Erfindungsgemäß eignen sich alle jenen bekannten Mikroorganismen, die aufgrund aktiver Dehydrogenasen zu oxidieren vermögen und Glycerin als Substrat erkennen. Vorallem die Gattungen Acetobacter und Gluconobacter sind hierfür einsetzbar, insbesondere Gluconobacter. Unter den Gluconobacter-Arten ist vorzugsweise Gluconobacter oxydans geeignet. Beispiele für allgemein zugängliche Gluconobacter oxydans Stämme sind die Stämme ATCC 621(DSM50049), DSM2343, ATCC 9324, ATCC23771 und DSM2003.

Die Mikroorganismen können vorteilhafterweise in üblichen für diese Zwecke geeigneten Kulturmedien vorkultiviert werden. Das eigentliche erfindungsgemäße Fermentationsmedium setzt sich qualitativ aus üblichen Bestandteilen zusammen. Vorteilhaft für das beanspruchte Verfahren hat sich beispielsweise der Einsatz von Hefeextrakt (0.3 - 0.7 %) (anstelle von Maisquellwasser), Glucose (0.5 - 1 %) und Magnesiumchlorid (0.1 - 0.2 %) erwiesen. Eine Anfangskonzentration von Calciumchlorid von 0.1 - 0.3 % ist ebenfalls günstig.

Erfindungsgemäß wird nun der pH-Wert des Mediums konstant bei einem bestimmten pH-Wert zwischen 3.8 und 4.8 gehalten. Vorzugsweise wird ein pH-Wert von 4.0 und 4.5, insbesondere aber 4.2 eingestellt. Dieser für die Umsetzung günstige pH-Wertbereich ist überraschend niedrig im Vergleich zu den meisten bekannten Verfahren, insbesondere bei Einsatz von Gluconobacter oxydans. Die Einstellung erfolgt kontinuierlich und über die gesamte Fermentationszeit durch Titration mit Calciumhydroxyd. Die geeignete Calciumhydroxyd-Suspension besteht aus handelsüblicher Kalkmilch, welcher zusätzlich Calciumoxid beigesetzt werden kann, so daß ein Gehalt an Calciumhydroxyd von mindestens 20 % vorliegt. Auch deutlich höhere Gehalte an Calciumhydroxid sind geeignet.

Die Fermentationstemperatur liegt erfindungsgemäß zwischen 28 und 34 °C, vorzugsweise zwischen 30 und 32 °C. Die Fermentationsbrühe wird in üblicher Weise mit Luft oder Sauerstoff belüftet.

In einer besonders vorteilhaften Ausführungsform des erfindungsgemäßen Verfahrens wird der Gyceringehalt in der Reaktionssuspension konstant bei 1 - 3 % (w/w). gehalten. Dies erfolgt durch kontinuierliches Zufüttern von Glycerin. Der jeweilige Glyceringehalt in der Reaktionssuspension wird nach allgemein bekannten Methoden bestimmt, beispielsweise durch Probenentnahme und Glycerinbestimmung mittels chromatographischer Bestimmungen.

Das erfindungsgemäße Verfahren liefert ausgezeichnete Ausbeuten an Dihydroxyaceton. So werden bereits in ca. 30 h 200 bis 260, durchschnittlich etwa 220 g/l, an Produkt ins Fermentationsmedium freigesetzt. Dabei werden 96 - 98 % des vorhandenen Glycerins umgesetzt. Es wird eine mittlere Produktivität von 6.8 bis 7.1 g/l h erreicht. Besonders vorteilhaft ist, daß die maximale Produktivität schon sehr früh nach 6 bis 10 h Fermentation erreicht wird. Sie beträgt für diesem Zeitraum 17 bis 20 g/l h. Somit werden die diesbezüglichen Werte des Standes der Technik übertroffen. Das Zufüttern von Substrat (Glycerin) bewirkt unter anderm, daß die Ausbeuten bereits in einer frühen Fermentationsphase erzielt werden.

Das gebildete Dihydroxyaceton kann aus der Fermentationsbrühe nach Standardmethoden isoliert und aufgereinigt werden.

Das erfindungsgemäße Verfahren ist hervorragend geeignet auch im großtechnischen Maßstab eingesetzt zu werden.

### Beispiel 1:

Mikroorganismen des Stammes *Gluconobacter oxydans* wurden nach Sandardverfahren vorkultiviert (Yamada et al. 1978, J. Ferment. Technol. 56, 29).

In ein 5000-l-Fermenter wurde ein Medium folgender Zusammensetzung eingebracht:

| *Gluconobacter oxydans* ATCC 621 (2.5 g Trockenmasse/l) | |
|---|---|
| Hefeextrakt | 5 g/l |
| Glucose | 5 g/l |
| Ammoniumsulfat | 2 g/l |
| Magnesiumsulfat (7-Hydrat) | 1 g/l |
| Calciumchlorid | 3 g/l und |
| Glycerin | 20 g/l |

Der Ausgangs-pH wurde mit Phosphorsäure auf 4.2 und die Fermentationstemperatur zwischen 30 und 32 °C eingestellt. Das Medium wurde während der gesamten Fermentation durch Einleiten von Luft belüftet. Der Glycerinverbrauch wurde durch etwa halbstündige Probenentnahme bestimmt und neues Glycerin durch Einrühren hinzugegeben, so daß wiederum eine Glycerinkonzentration von etwa 20 g/l im Fermentationsansatz vorlag. Ebenso wurde durch pH-Messung und gegebenenfalls permanentes automatisches Hinzutitrieren von Calciumhydroxid-Suspension (20%ig) der pH-Wert auf 4.5 fixiert.

Nach etwa 5 h wurden Proben entnommen und der Gehalt an Dihydroxyaceton nach Standardmethoden bestimmt. Es hatten sich etwa 25 g/l Produkt gebildet. Nach 10 Stunden wurde ein Dihydroxyacetongehalt von bereits ca. 100 g/l im Medium gefunden. In diesem Zeitraum wurde die stärkste Produktivität des Verfahrens (18.5 g/l h Dihydroxyaceton) ermittelt. Nach 20 h Fermentation ließ sich ein Gehalt von etwa 170 g/l Produkt feststellen. nach 31 h wurden 219 g/l Dihydroxyaceton gemessen. Weiteres fermentieren erbrachte nur noch geringfügig höhere Mengen (bis 260 g/l).

### Beispiel 2:

Analog Beispiel 1 wurde eine Fermentation durchgeführt, bei der die kontinuierliche Zugabe von Calciumhydroxid-Suspension weggelassen wurde, so daß der pH-Wert nicht nachreguliert wurde. Nach 10 h Fermentation wurde ein Dihydroxyaceton-Gehalt von etwa 75 g/l ermittelt. Nach 31 h wurde der entsprechende Gehalt zu etwa 190 g/l bestimmt. Die Produktivität im Zeitraum zwischen 5 und 15 h betrug maximal 13 g/l h. Bei dem verwendeten 5.000-l-Fermenter sind dies etwa 20 kg/h weniger Ausbeute an Dihydroxyaceton als in Beispiel 1.

### Beispiel 3:

Analog Beispiel 1 wurde eine Fermentation durchgeführt, bei der die konstante Zufütterung von Substrat (Glycerin, zwischen 1 - 3 %) ersetzt wurde durch eine einmalige Glyceringabe (130 g/l) zu Beginn der Fermentation. Nach 80 h Fermentation wurden 106 g/l Dihydroxyaceton gebildet.

### Beispiel 4:

Es wurde eine Fermentation analog Beispiel 1 durchgeführt, wobei anstelle von *Gluconobacter oxydans* der Mikroorganismuns *Acetobacter xylinum* eingesetzt wurde. Nach 28 h Fermentation konnten etwa 975 kg Dihydroxyaceton (195 g/l) aus dem Medium isoliert werden.

### Beispiel 5:

Es wurde eine Fermentation analog Beispiel 1 durchgeführt, wobei das Medium 2 g/l Hefeextrakt, 2 g/l Maisquellwasser und 2 g/l Glucose enthalt. Nach 30 h Fermentation betrug der Gehalt an Dihydroxyaceton im Medium 198 g/l.

### Beispiel 6:

Es wurde eine Fermentation analog Beispiel 1 bei einem pH-Wert von 3.8 durchgeführt. Nach 31 h Fermentation betrug der Gehalt an Dihydroxyaceton im Medium 179 g/l.

### Beispiel 7:

Es wurde eine Fermentation analog Beispiel 1 bei einem pH-Wert von 4.8 durchgeführt. Nach 31 h Fermentation betrug der Gehalt an Dihydroxyaceton im Medium 205 g/l.

## Patentansprüche

1. Verfahren zur Herstellung von Dihydroxyaceton durch Dehydrierung von Glycerin mittels dehydogenaseaktiver Mikroorganismen, dadurch gekennzeichnet, daß man die Reaktion durch Titration mittels Calciumhydroxid-Suspension bei einem konstant gehaltenen pH-Wert, ausgewählt in einem Bereich zwischen 3.8 und 4.8, durchführt.

2. Verfahren nach Anspruch 1, dadurch gekennzeichnet, daß die Reaktion in einem pH-Bereich zwischen 4.0 und 4.5 durchgeführt wird.

3. Verfahren nach Anspruch 1 oder 2, dadurch gekennzeichnet, daß die Reaktion bei einem pH-Wert von 4.2 durchgeführt wird.

4. Verfahren nach einem der Ansprüche 1 bis 3, dadurch gekennzeichnet, daß der Glyceringehalt durch Zudosierung konstant zwischen 1 und 3 % (w/w) gehalten wird.

5. Verfahren nach einem der Ansprüche 1 bis 4, dadurch gekennzeichnet, daß als Mikroorganismus ein Bakterium der Gattung *Gluconobacter* eingesetzt wird.

6. Verfahren nach Anspruch 5, dadurch gekennzeichnet, daß der Mikroorganismus *Gluconobacter oxidans* eingesetzt wird.

## Claims

1. Process for the preparation of dihydroxyacetone by dehydrogenation of glycerol by means of microorganisms having active dehydrogenase, characterized in that the reaction is carried out by titration by means of calcium hydroxide suspension at a pH which is kept constant and is selected in a range between 3.8 and 4.8.

2. Process according to Claim 1, characterized in that the reaction is carried out in a pH range between 4.0 and 4.5.

3. Process according to Claim 1 or 2, characterized in that the reaction is carried out at a pH of 4.2.

4. Process according to one of Claims 1 to 3, characterized in that the glycerol content is kept constant between 1 and 3% (w/w) by metering in.

5. Process according to one of Claims 1 to 4, characterized in that the microorganism used is a bacterium of the genus *Gluconobacter*.

6. Process according to Claim 5, characterized in that the microorganism *Gluconobacter oxidans* is used.

## Revendications

1. Procédé pour la préparation de la dihydroxyacétone par déshydrogénation du glycérol au moyen de micro-organismes à activité déshydrogénase, caractérisé en ce que l'on effectue la réaction, par titrage à l'aide d'une suspension d'hydroxyde de calcium, à un pH maintenu constant, choisi dans un intervalle compris entre 3,8 et 4,8.

2. Procédé selon la revendication 1, caractérisé en ce que l'on effectue la réaction dans un intervalle de pH compris entre 4,0 et 4,5.

3. Procédé selon la revendication 1 ou 2, caractérisé en ce que l'on effectue la réaction à un pH de 4,2.

4. Procédé selon l'une des revendications 1 à 3, caractérisé en ce que la teneur en glycérol est maintenue constante entre 1 et 3 % (p/p) par addition réglée.

5. Procédé selon l'une des revendications 1 à 4, caractérisé en ce que l'on utilise comme micro-organisme une bactérie appartenant au genre *Gluconobacter*.

6. Procédé selon la revendication 5, caractérisé en ce que l'on utilise le micro-organisme *Gluconobacter oxidans*.
